Europäisches Patentamt

European Patent Office

Office européen des brevets

(19)

(11) Veröffentlichungsnummer: **0 316 787**

**A2**

## (12) EUROPÄISCHE PATENTANMELDUNG

(21) Anmeldenummer: 88118792.6

(51) Int. Cl.⁴: **C07C 155/06**

(22) Anmeldetag: **11.11.88**

(30) Priorität: **19.11.87 DE 3739149**

(43) Veröffentlichungstag der Anmeldung:
**24.05.89 Patentblatt 89/21**

(84) Benannte Vertragsstaaten:
**AT BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Zeidler, Adolf, Dr.**
**Ungsteiner Strasse 47**
**D-6700 Ludwigshafen(DE)**
Erfinder: **Thoemel, Frank, Dr.**
**Leberstrasse 17**
**D-6940 Weinheim(DE)**
Erfinder: **Scholz, Herbert, Dr.**
**Im Finkenschlag 16**
**D-6730 Neustadt(DE)**
Erfinder: **Krader, Thomas, Dr.**
**B 4,15**
**D-6800 Mannheim 1(DE)**
Erfinder: **Woerz, Otto, Dr.**
**Friedrich-Pietzsch-Strasse 10**
**D-6701 Friedelsheim(DE)**
Erfinder: **Koob, Knut, Dr.**
**Lorscher Strasse 7**
**D-6704 Mutterstadt(DE)**
Erfinder: **Thiele, Heino, Dr.**
**An der Froschlache 23**
**D-6700 Ludwigshafen(DE)**

(54) **Verfahren zur Herstellung von Mono oder Bis-Dithiocarbaminsäuren bzw. deren Salzen und deren Folgeprodukten.**

(57) Verfahren zur Herstellung von Mono- oder Bis-Dithiocarbaminsäuren bzw. deren Salzen der allgemeinen Formel I

$$R^1R^2N\text{-}CS\text{-}S^{\ominus}A^{\oplus} \quad \text{(I)},$$

in der $R^1$ und $R^2$ Wasserstoff, einen $C_1$- bis $C_4$-Alkylrest oder zusammen eine $C_2$-$C_4$-Alkylenkette bilden und $R^2$ darüber hinaus einen Rest der Struktur $R^1N\text{-}CS\text{-}S^{\ominus}A^{\oplus}$ über eine $C_2$-$C_4$-Alkylenkette verknüpft und $A^{\oplus}$ ein Ammoniumion oder ein von Ammonium abgeleitetes Kation bedeuten, durch Umsetzung eines entsprechenden Amins oder Alkylenamins mit Schwefelkohlenstoff, wobei man die Umsetzung bei einem solchen Druck vornimmt, daß eine Umsetzungstempratur von wenigstens 50°C erreicht wird.

EP 0 316 787 A2

## Verfahren zur Herstellung von Mono- oder Bis-Dithiocarbaminsäuren bzw. deren Salzen und deren Folgeprodukten

Die vorliegende Erfindung betrifft ein verbessertes Verfahren zur Herstellung von Mono- oder Bis-Dithiocarbaminsäuren bzw. deren Salzen der allgemeinen Formel I

$$R^1R^2N\text{-}CS\text{-}S\text{-}^\ominus A^\oplus \quad (I),$$

in der $R^1$ und $R^2$ Wasserstoff, einen $C_1$- bis $C_4$-Alkylrest oder zusammen eine $C_2$-$C_4$-Alkylenkette bilden und $R^2$ darüber hinaus einen Rest der Struktur $R^1N\text{-}CS\text{-}S^\ominus A^\oplus$ über eine $C_2$-$C_4$-Alkylenkette verknüpft und $A^\oplus$ ein Ammoniumion oder ein von Ammonium abgeleitetes Kation bedeuten, durch Umsetzung eines entsprechenden Amins oder Alkylenamins mit Schwefelkohlenstoff.

Dithiocarbaminsäuren bzw. deren Salze sind Zwischenprodukte z.B. für die Herstellung von Pflanzenschutzmitteln wie Metamsodium, Dazomet oder Metiram (vgl. Ullmanns Encyklopädie der techn. Chemie, 4. Aufl., Bd. 10, 1975, S. 167-180 und dort zitierte Literatur).

Metamsodium (Natrium-methyldithiocarbamat) und Dazomet (3,5-Dimethyl-tetrahydro-1,3,5-thiadiazinthion-2, vgl. Römpps Chemie-Lexikon, 8. Aufl., 1981, S. 872) sind handelsübliche Bodendesinfektionsmittel; Metiram, eine Mischfällung aus dem Ammoniakkomplex von Zink-[N,N'-ethylen-bis-(dithiocarbamat)] und N,N'-Polyethylen-bis-(thiocarbamoyl)-disulfid, ist ein Fungizid-Wirkstoff.

Die Umsetzung z.B. von Methylamin bzw. dessen wäßriger Lösung mit Schwefelkohlenstoff und Zersetzung des intermediär gebildeten Methylammoniumsalzes der Methyldithiocarbaminsäure mit (Natron)lauge liefert Metamsodium, wobei das freigesetzte Methylamin mit weiterem Schwefelkohlenstoff zu weiterem Dithiocarbamat reagiert, nach dem nachstehenden, zweistufigen Schema:

(1) $2\ H_3CNH_2 + CS_2 \rightarrow H_3CNH\text{-}CS\text{-}S^\ominus\ H_3NCH_3^\oplus$
(2) $H_3CNH\text{-}CS\text{-}S^\ominus\ H_3CNH_3^\oplus + NaOH \rightarrow H_3CNH\text{-}CS\text{-}S^\ominus\ Na^\oplus$

Entsprechend verläuft die Umsetzung mit Ethylendiamin und Ammoniak, die zu einem Vorprodukt für Metiram führt.

Die Forderung, daß das Zielprodukt möglichst frei von Ausgangsstoffen und Nebenprodukten sein soll, wird praktisch dadurch verwirklicht, daß man die Umsetzung bei möglichst niedriger Temperatur (z.B. bis 40°C) vornimmt; dabei liegt die Vorstellung zugrunde, daß das wesentliche Nebenprodukt, N,N'-Dimethylthioharnstoff, durch thermische Spaltung des vorerwähnten Methylammoniumsalzes entsteht. Seine Konzentration soll 0,1 % im Zielprodukt nicht überschreiten.

Außerdem wird die Umsetzung tatsächlich entsprechend dem angegebenen zweistufigen Schema vorgenommen, d.h. Natronlauge erst zugegeben, wenn die Salzbildung nach Gleichung (1) stattgefunden hat, wobei allerdings die Gesamtmenge an Schwefelkohlenstoff von vorneherein zugegen sein kann, um eine hinreichende Umsetzungsgeschwindigkeit zu erzielen. Die Natronlauge darf im übrigen nur nach und nach zugegeben werden, wobei ein pH-Wert von 9,5 nicht überschritten werden darf.

Dieses Verfahren, das analog im Prinzip auch bei der Herstellung der anderen, oben angegebenen Wirkstoffe angewendet wird, ist wegen seiner geringen Geschwindigkeit und schwieriger Regelbarkeit nachteilig.

Es wurde nun gefunden, daß die Herstellung von Mono- oder Bis-Dithiocarbaminsäuren bzw. deren (Ammonium)salzen, der allgemeinen Formel

$$R^1R^2N\text{-}CS\text{-}S^\ominus\ A^\oplus$$

in der $R^1$ und $R^2$ Wasserstoff einen $C_1$- bis $C_4$-Alkylrest oder zusammen eine $C_2$-$C_4$-Alkylenkette bilden und $R^2$ darüber hinaus einen Rest der Struktur $R^1N\text{-}CS\text{-}S^\ominus A^\oplus$ über eine $C_2$- bis $C_4$-Alkylenkette verknüpft und $A^\oplus$ ein Ammoniumion oder ein von Ammonium abgeleitetes Kation bedeuten, durch Umsetzung eines entsprechenden ein- oder mehrwertigen Amins mit Schwefelkohlenstoff, mit dem Vorteil wesentlich höherer Geschwindigkeit und - überraschenderweise - geringerer Bildung von Nebenprodukten durch eine relativ kleine Veränderung der Bedingungen verbessert werden kann, wenn man die Umsetzung bei einem solchen Druck vornimmt, daß eine Umsetzungstempratur von wenigstens 50°C erreicht wird.

Erfindungsgemäß wird die beabsichtigte Verbesserung dadurch erzielt, daß man die Umsetzung wenigstens zeitweise bei einer Temperatur von etwa 50 bis 80, vorteilhaft 55 bis 70°C vornimmt. Hierzu muß lediglich geringfügig überatmosphärischer Druck, nämlich etwa 2 bis 4 bar angewendet werden; der Schwefelkohlenstoff, der in der wäßrigen Phase nur wenig löslich ist, würde sonst verdampfen.

Das Wesen der Erfindung besteht somit darin, eine Reaktionstemperatur zu ermöglichen, die wenig bis mäßig oberhalb der Siedetemperatur des Reaktionsgemisches (praktisch der Siedetemperatur von 46°C des Schwefelkohlenstoffs) bei atmosphärischem Druck liegt. Dadurch läßt sich die an sich langsam ablaufende Umsetzung, die zu einem Zwischenprodukt der Formel I führt, soweit beschleunigen, daß sie nicht mehr geschwindigkeitsbestimmend ist.

Als Ausgangsstoffe können primäre oder sekundäre Amine oder Diamine verwendet werden. Beispielsweise seien folgende Amine aufgeführt: Methyl-, Dimethyl-, Ethyl-, Diethyl-, Propyl-, Din-Propyl-, Isopropyl-, Butyl- oder Di-n-Butylamin, wobei Methylamin von besonderem Interesse zur Herstellung von Metamsodium oder Dazomet ist. Als Diamine seien beispielsweise Ethylendiamin, 1,2-Propylendiamin oder 1,3-Diaminopropan genannt, wobei Ethylendiamin von besonderem Interesse zur Herstellung von Metiram ist.

Die stöchiometrischen Verhältnisse entsprechen dem üblichen, d.h. eventueller Überschuß an einem Reaktionsteilnehmer wird jeweils durch verfahrenstechnische Maßnahmen wiederverwendbar gemacht, also zurückgeführt. Im allgemeinen wird ein geringer Überschuß an Schwefelkohlenstoff vorzuziehen sein. Das erhaltene Zwischenprodukt kann aus der wäßrigen Lösung für sich gewonnen werden, z.B. durch Eindampfen. Praktisch geht man jedoch zweckmäßig so vor, daß man die zweite Umsetzung unmittelbar anschließt bzw. durch Laugenzugabe parallel ablaufen läßt.

Die Reaktionsgeschwindigkeit wird unter diesen Umständen durch die Zugabe der Lauge gesteuert, d.h. die Umsetzung zum Wirkstoff läuft in dem Maße ab, wie Lauge zugegeben wird. Die Geschwindigkeit der Zugabe wird nur durch die mögliche Wärmeabfuhr (die Umsetzung verläuft exotherm) begrenzt. Die Weiterverarbeitung des Zwischenproduktes Methyldithiocarbaminsäure in Form das Methylammoniumsalzes zu Dazomet (Tetrahydro-3,5-dimethyl-2H-1,3,5-thiazin-thion(2)) kann in an sich bekannter Weise durch Umsetzung Formaldehyd und weiterem Methylamin vorgenommen werden.

Zusätzlich kann auch die Einhaltung eines pH-Wertes von 9,5 oder weniger als Regelungsgröße für die Laugendosierung gewählt werden.

Der erhaltene Wirkstoff kann unmittelbar aus dem Reaktionsgemisch durch Eindampfen, Sprühtrocknen u.ä. gewonnen werden, soweit nicht ohnehin die wäßrige Lösung Anwendung findet oder handelsüblich ist.

Beispiel 1

Es werden 600 ml Wasser und 660 ml 40 % wäßrige Monomethylaminlösung vorgelegt. Unter Rühren und Stickstoffschutz pumpt man 230 ml Schwefelkohlenstoff so zu, daß die Temperatur nicht über 70°C steigt (Kühlung). Der Druck steigt dabei auf 2,1 bar an. Es wird eine Stunde bei 70°C nachgerührt.

Das entstandene Methylammoniumsalz der Methyldithiocarbaminsäure kann unmittelbar als Zwischenprodukt für die Synthese von Metamfluid oder Dazomet verwendet werden.

Beispiel 2

In einem 2 l fassenden, druckfesten 5-Halskolben, ausgestattet mit Rührwerk, Thermometer, pH-Elektrode, Kühler und Dosiereinrichtung werden 219,8 g einer 40 %iges wäßriges Monomethylamin und 97,5 g Wasser vorgelegt. Dies entspricht dann einer 27,7 %igen Monomethylaminlösung oder 87,9 g Monomethylamin. Die Lösung wird im Wasserbad auf 38°C gebracht und bei konstanter Temperatur innerhalb von 30 Minuten unter Rühren mit 2113 g Schwefelkohlenstoff versetzt, welcher über eine Dosierpumpe zugefördert wird.

Danach wird auf 70°C aufgeheizt, wodurch sich ein Überdruck von 2,25 bar einstellt. Es werden 229 g 50 %ige Natronlauge mit einer geeigneten Pumpe so dosiert, daß der pH-Wert nicht über 9,5 ansteigt. Hierzu wird die Pumpe über das Ausgangssignal einer pH-Meßelektrode gesteuert und so ein Überschreiten des Grenzwertes vermieden. Die Temperatur wird durch das Wasserbad auf 70°C gehalten. Der Dosiervorgang dauert 15 min.

Nach Beendigung der Zugabe wird auf Raumtemperatur abgekühlt - dabei fällt der Druck auf atmosphärischen Druck ab - und eine Probe entnommen. Der Gehalt an unerwünschtem Nebenprodukt (Dimethylthioharnstoff) liegt unter 0,1 %, die Lösung hat eine rotgelbe Farbe und die Ausbeute entspricht der stöchiometrischen Umwandlung der Rohstoffe zum gewünschten Produkt.

Beispiel 3

Ähnlich der Verfahrensweise in Beispiel 1 wurden 220 g 40 %ige Methylaminlösung und 97,5 g Wasser vorgelegt und auf 40°C aufgewärmt. Anschließend wurden 224,3 g Schwefelkohlenstoff bei konstanter Temperatur nach und nach zugesetzt.

Ohne die Temperatur zu verändern, wurden nun 182 g 50 %ige Natronlauge innerhalb von 40 min zugesetzt. Kontrolle und Einhaltung des pH-Wertes geschah erfolgte wie in Beispiel 1. Nach Entnahme einer Probe zur Bestimmung des Umsatzes wurde auf 70°C aufgeheizt und zur Vervollständigung des Umsatzes weitere 47,8 g 50 %ige Natronlauge innerhalb von 18 min zugepumpt.

Die Mischung wurde wieder auf Raumtemperatur abgekühlt und analytisch untersucht. Entsprechend den eingesetzten Mengen wurde die stöchiometrische Ausbeute erreicht und der Nebenproduktgehalt (Dimethylthioharnstoff) betrug wiederum weniger als 0,1 %. Die Farbe des Reaktionsgemisches war gelborange mit leicht grünlichem Farbton.

Beispiel 4

Es werden 1 l Wasser, 165 ml Ethylendiamin und 360 ml 25 % Ammoniakwasser vorgelegt. Unter Rühren und Stickstoffschutz pumpt man 290 ml Schwefelkohlenstoff so zu, daß eine Temperatur von 70 °C nicht überschritten wird (Kühlung). Der Druck steigt auf 2,2 bar an. Man rührt 30 Minuten bei 70 °C nach.

Das Ammoniumsalz dient als Zwischenprodukt für die Synthese von Metiram.

**Ansprüche**

1. Verfahren zur Herstellung von Mono- oder Bis-Dithiocarbaminsäuren bzw. deren Salzen der allgemeinen Formel I

$$R^1R^2N\text{-}CS\text{-}S^\ominus A^\oplus \qquad (I),$$

in der $R^1$ und $R^2$ Wasserstoff, einen $C_1$- bis $C_4$-Alkylrest oder zusammen eine $C_2$-$C_4$-Alkylenkette bilden und $R^2$ darüber hinaus einen Rest der Struktur $R^1N\text{-}CS\text{-}S^\ominus A^\oplus$ über eine $C_2$-$C_4$-Alkylenkette verknüpft und $A^\oplus$ ein Ammoniumion oder ein von Ammonium abgeleitetes Kation bedeuten, durch Umsetzung eines entsprechenden Amins oder Alkylenamins mit Schwefelkohlenstoff, dadurch gekennzeichnet, daß man die Umsetzung bei einem solchen Druck vornimmt, daß eine Umsetzungstemperatur von wenigstens 50 °C erreicht wird.

2. Verfahren zur Herstellung von Natriummethyldithiocarbamat (Metam-Sodium) durch Umsetzung von Methylamin und Schwefelkohlenstoff in Gegenwart von Natronlauge, wobei zunächst Schwefelkohlenstoff in wäßriges Methylamin eingetragen und das bei der Spaltung des intermediär gebildeten Methylammoniumsalzes der Methyldithiocarbaminsäure mit Lauge freigesetzte Methylamin von überschüssigem Schwefelkohlenstoff gebunden wird, dadurch gekennzeichnet, daß die Umsetzung bei einem solchen Druck vorgenommen wird, daß eine Umsetzungstemperatur von wenigstens 50 °C erreicht wird.

3. Verfahren zur Herstellung von Ammoniumethylenbisdithiocarbamat durch Umsetzen von Ethylendiamin und Schwefelkohlenstoff in wäßrig-ammoniakalischer Lösung, dadurch gekennzeichnet, daß die Umsetzung bei einem solchen Druck vorgenommen wird, daß eine Umsetzungstemperatur von wenigstens 50 °C erreicht wird.

4. Verfahren nach einem der Ansprüche 1 bis 3, dadurch gekennzeichnet, daß ein Druck von etwa 2 bis 4 bar, entsprechend etwa 50 bis 80 °C eingestellt wird.